(19) Europäisches Patentamt
European Patent Office
Office européen des brevets

(11) EP 1 754 471 A1

(12) **EUROPÄISCHE PATENTANMELDUNG**

(43) Veröffentlichungstag:
**21.02.2007 Patentblatt 2007/08**

(51) Int Cl.:
*A61K 9/50* (2006.01)   *A61K 9/20* (2006.01)

(21) Anmeldenummer: 05017958.9

(22) Anmeldetag: **18.08.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **ORAMON-Arzneimittel GmbH & Co. KG 88471 Laupheim (DE)**

(72) Erfinder:
• **Braun, Michael, Dr.**
  **88471 Laupheim (DE)**
• **Neuer, Klaus**
  **88471 Laupheim (DE)**

• **Beckert, Thomas, Dr.**
  **88471 Laupheim (DE)**
• **Heinz, Carmen**
  **88471 Laupheim (DE)**
• **Brunner, Melanie**
  **88471 Laupheim (DE)**

(74) Vertreter: **Schwabe - Sandmair - Marx Patentanwälte P.O. Box 86 02 45 81629 München (DE)**

Bemerkungen:
Geänderte Patentansprüche gemäss Regel 86 (2) EPÜ.

(54) **Verwendung einer quellbaren Zwischenschicht zur Steuerung des Freisetzungsprofils einer festen Arzneimittelformulierung zur peroralen Anwendung und diese enthaltende Arzneimittelformulierung**

(57)   Die vorliegende Erfindung betrifft die Verwendung einer quellbaren Zwischenschicht, umfassend ein quellbares Material, ausgewählt aus der Gruppe, bestehend aus Stärke, Stärkederivaten wie vorgelierte Stärke, Na-Carboxyethylstärke, Na-Stärkeglykolat, Cellulose, Celluloseethern wie HPMC, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, in einer verzögert freisetzenden, festen Arzneimittelformulierung zur peroralen Anwendung, welche Formulierung ein einen Wirkstoff enthaltendes Substrat, die quellbare Zwischenschicht und eine retardierende Überzugsschicht aufweist, zur Einstellung eines Freisetzungsprofils des Wirkstoffes, welches gegen Ende eine im Wesentlichen vollständige Freisetzung gestattet, und die entsprechende verzögert freisetzende, feste Arzneimittelformulierung zur peroralen Anwendung.

EP 1 754 471 A1

**Beschreibung**

Die vorliegende Erfindung betrifft die Verwendung einer quellbaren Zwischenschicht aus einem quellbaren Material zur Einstellung eines Freisetzungsprofils einer verzögert freisetzenden, festen Arzneimittelformulierung zur peroralen Anwendung sowie verzögert freisetzende, feste Arzneimittelformulierungen zur peroralen Anwendung, enthaltend eine solche quellbare Zwischenschicht.

**Stand der Technik**

Verzögert freisetzende, feste Arzneimittelformulierungen zur oralen Verwendung sind wohl bekannt. Sie werden unter anderem in der Schmerztherapie, bei Herz-Kreislauferkrankungen oder Erkrankungen des zentralen Nervensystems weit verbreitet verwendet. Zweck der Verabreichung dieser verzögert, d.h. langsam freisetzenden Formulierungen besteht darin, eine länger anhaltende pharmakologische Wirkung nach Verabreichung zu erzielen als dies durch Verabreichung unmittelbar bzw. sofort freisetzender Dosisformen möglich ist. Solche längere Zeitspannen der pharmakologischen Wirkung bieten zahlreiche therapeutische Vorteile, die sich mit den herkömmlichen, unmittelbar freisetzenden Zubereitungen nicht erzielen lassen. Somit kann bspw. die Therapie auch ohne Unterbrechung des Schlafs des Patienten fortgesetzt werden. Weiterhin erhöht eine Verringerung der täglich einzunehmenden Dosen die Patienten-Compliance beträchtlich.

Bei Verabreichung sofort freisetzender, schnell wirkender Arzneiformen muss die Verabreichung in regelmäßigen, kurzen Intervallen von z.B. 4 bis 6 Stunden erfolgen, um bestimmte Wirkstofflevel im Plasma des Patienten aufrecht zu erhalten. Spitzen dieses Blutlevels aber auch ein zu weitgehendes Absinken sind zu vermeiden, was im Hinblick auf unterschiedliche Sekretion, metabolische Inaktivierung, oder auch die Patienten Compliance schwierig sein kann. Konkret ist daher z.B. auf eine sehr regelmäßige Einnahme zu achten. Dies ist oftmals jedoch nicht gewährleistet.

Die verzögerte Freisetzung hilft bei der Überwindung dieser Probleme. Hierbei wird von einer einmal eingenommenen Dosis über längere Zeit hinweg kontinuierlich Wirkstoff freigesetzt, was zu entsprechenden Plasmaspiegeln führt. Nach dem Funktionsprinzip können u.a. osmotische, erodierende und Diffusionssysteme unterschieden werden. Die Diffusionssysteme wiederum können matrix- oder membrangesteuert sein. Auch Mischformen sind möglich. Die verzögerte Freisetzung membrangesteuerter Diffusionssysteme wird in der Regel durch Auf trag einer Beschichtung aus einem unlöslichen Polymeren bspw. aus Ethylcellulose erzielt, der weitere Bestandteile wie bspw. Weichmacher zugesetzt werden können. Entsprechende feste, verzögert freisetzende, orale Dosisformen sind bspw. in der EP-A-1 243 269 sowie der EP-A-1 419 766 beschrieben.

Beide Dokumente beschreiben verzögert freisetzende Opioidformulierungen, bei denen die verzögerte Freisetzung üblicherweise durch Überschichten eines den Wirkstoff enthaltenden Substrats oder (Pellet)-Kerns mit einer ausreichenden Menge einer wässrigen Dispersion eines unlöslichen Polymeren wie Ethylcellulose, Acrylpolymer, Schellack, Zein, einem hydrophoben Wachs und Mischungen davon zum Erhalt eines Überzugs erzielt wird. Dieser Beschichtung können Hilfsstoffe wie Weichmacher (bspw. Citratester, Phthalatester oder Sebacatester), Farbstoffe, Porenbildner (wie Hydroxypropylmethylcellulose) oder andere hydrophile Polymere, die Erosion fördernde Mittel (wie Stärke) oder ähnliches zugesetzt werden.

Weiterhin können die Arzneimittelformulierungen gemäß EP 1243 269 und EP 1 419 766 zwischen dem wirkstoffhaltigen Substrat und der die verzögerte Freisetzung bewirkenden Überzugsschicht eine Sperrschicht enthalten. Diese dient im Regelfall zur Verbesserung der Wirkstoffstabilität durch Trennung des Wirkstoffes von der die verzögerte Freisetzung steuernden Überzugsschicht. Vorzugsweise enthält eine solche Zwischenschicht Hydroxypropylmethylcellulose niedriger Viskosität (z.B. Pharmacoat 603). Diese Sperrschicht soll jedoch die Freisetzungsgeschwindigkeit des Produktes nicht beeinflussen.

Die aus dem Stand der Technik bekannten, verzögert freisetzenden, festen Arzneimittelformulierungen zur oralen Verabreichung weisen den Nachteil auf, dass die Freisetzungseigenschaften derselben neben den Polymereigenschaften und der Art und dem Anteil der verwendeten Hilfsstoffe wesentlich von der Dicke der verzögert freisetzenden Beschichtung aus hydrophobem Polymer abhängig sind. Wird diese sehr dick gewählt, ist oftmals gegen Ende des Untersuchungszeitraums, der üblicherweise zwischen 8 und 12 Stunden liegt, eine gegenüber dem Beginn deutlich geringere Wirkstofffreisetzung / Zeiteinheit, d.h. eine Abflachung des Freisetzungsprofils zu beobachten. Häufig kann deshalb keine vollständige Wirkstofffreisetzung erzielt werden. Zudem kann die anfängliche Freisetzungsrate zu gering ausfallen. Wird dementsprechend die Dicke der Beschichtung verringert, ist die Freisetzung zu Beginn des Untersuchungszeitraums (bpsw. bis zu 1 Stunde nach Verabreichung) oft zu schnell. In anderen Worten zeigen die Freisetzungsprofile, die sich mit Hilfe solcher verzögert freisetzender Beschichtungen erzielen lassen, gegen Ende des Untersuchungszeitraums einen zu flachen Verlauf.

Die oben genannten Dokumente schlagen zur Lösung dieses Problems die Verwendung bestimmter, die Freisetzung modifizierender Mittel wie Porenbildner in Form hydrophiler löslicher Polymere, langkettiger abbaubarer Kohlenwasserstoffe wie Fettsäuren, Fettalkoholen, Glycerinestern derselben, Polyalkylenglykole, Stärken, Gummen, Polyester oder ähnliche in der Überzugsschicht vor. Das hierdurch erzielte Freisetzungsprofil weist jedoch immer noch eine Freisetzung von etwa 12,5 % bis etwa 42,5 % nach einer Stunde und mehr als etwa 60 % nach 8 Stunden gemessen über die USP Paddlemethode bei 100 rpm und 900 ml wässrigem Puffer (pH zwischen 1,6 und 7,2) bei 37°C auf. Damit wird nach wie vor keine im Wesentlichen vollständige Freisetzung am Ende des Freisetzungszeitraumes erzielt, ist eine wirkungsvolle Verzögerung der Freisetzung am Anfang jedoch teilweise schon erheblich beeinträchtigt. Die anfänglich unzulängliche Freisetzung bei dicken Beschichtungen kann durch die Verwendung von Mischungen verzögert freisetzender und sofort freisetzender Arzneiformen aufgefangen werden. Damit überlagern sich jedoch 2 Profile und muss das Verhältnis beider Formen sorgsam abgestimmt werden.

Die vorliegende Erfindung hat zur Aufgabe, diese und weitere Nachteile des Standes der Technik zu überwinden.

## Kurze Beschreibung der Erfindung

Die vorliegende Erfindung soll die obigen Probleme des Standes der Technik überwinden und insbesondere eine Steuerung bzw. Einstellung des Freisetzungsprofils einer festen, verzögert freisetzenden Arzneimittelformulierung zur peroralen Anwendung gestatten. Dies wird ermöglicht durch die Verwendung einer quellbaren Zwischenschicht, umfassend ein quellbares Material, ausgewählt aus der Gruppe, bestehend aus Stärke, Stärkederivaten wie vorgelierte Stärke, Na-Carboxyethylstärke, Na-Stärkeglykolat, Cellulose, Celluloseethern wie HPMC, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, in einer verzögert freisetzenden, festen Arzneimittelformulierung zur peroralen Anwendung, welche Formulierung ein einen Wirkstoff enthaltendes Substrat, die quellbare Zwischenschicht und eine retardierende Überzugsschicht aufweist, zur Einstellung eines Freisetzungsprofils des Wirkstoffes, welches gegen Ende eine im Wesentlichen vollständige Freisetzung gestattet.

Genauso betrifft die vorliegende Erfindung eine verzögert freisetzende, feste Arzneimittelformulierung zur peroralen Anwendung, welche Formulierung ein einen Wirkstoff enthaltendes-Substrat, eine quellbare Zwischenschicht und eine retardierende Überzugsschicht aufweist, worin die quellbare Zwischenschicht ein quellbares Material umfasst, das ausgewählt ist aus der Gruppe, bestehend aus Stärke, Stärkederivaten wie pregelatinisierter Stärke, Na-Carboxyethylstärke, Na-Stärkeglykolat, Cellulose, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, und in einer zur Einstellung eines Freisetzungsprofils des Wirkstoffes, welches gegen Ende eine im Wesentlichen vollständige Freisetzung gestattet, wirksamen Menge vorhanden ist.

## Genaue Beschreibung der Erfindung

Die vorliegende Erfindung basiert auf der Erkenntnis, dass sich das Freisetzungsprofil von verzögert freisetzenden, festen Arzneimittelformulierungen zur peroralen Anwendung nicht ausschließlich über die Wahl und Gestaltung der retardierenden Überzugsschicht beeinflussen lässt, sondern zusätzlich durch die Verwendung einer speziellen, nämlich quellbaren Zwischenschicht beeinflusst werden kann.

"Verzögert freisetzend" im Sinne der vorliegenden Anmeldung meint Arzneimittelformulierung, welche den in ihnen enthaltenen Wirkstoff in einer Zeitspanne von mehr als 6, vorzugsweise mehr als 8 und üblicherweise 8 bis 12 Stunden nach peroraler Verabreichung im Wesentlichen vollständig freisetzen. Die Freisetzung wird erfindungsgemäß nach dem USP Paddle oder Drehkörbchenverfahren gemessen, wie es in der US Pharmacopoiea XXII (1990) beschrieben ist. Hierbei wird eine Paddleapparatur mit Drehkörbchen gemäß Ph.Eur.2.9.3-1 verwendet. Dieses wird mit 100 rpm in 900 ml eines Phosphatpuffers, pH 6,4 gemäß USP, über die Messzeit gedreht und die in die Lösung abgegebene Menge an Wirkstoff bestimmt, wobei die Freisetzung in Prozent der in der Formulierung enthaltenen Gesamtmenge angegeben wird:

$$\text{Freigesetzte Menge} \times 100/\text{Gesamtmenge} = \%\ \text{Freisetzung}$$

Mit "im Wesentlichen vollständiger Freisetzung" ist erfindungsgemäß eine Freisetzung von mindestens 75 % der Gesamtmenge des in der Formulierung enthaltenden Wirkstoffes, vorzugsweise mindestens 85 % der Gesamtmenge des in der Formulierung enthaltenden Wirkstoffes gemeint. Mit "gegen Ende" der Freisetzung ist der Endpunkt der obigen Zeitspanne von mehr als 6 vorzugsweise 8 bis 12 h nach Verabreichung gemeint. Die "anfängliche" Freisetzung bezieht sich auf die Freisetzung innerhalb 1 h nach Verabreichung.

Die Verwendung der erfindungsgemäßen quellbaren Zwischenschicht in der verzögert freisetzenden, festen Arzneimittelformulierung zur peroralen Anwendung, welche ein einen Wirkstoff enthaltendes Substrat, die quellbare Zwischenschicht und eine retardierende Überzugsschicht aufweist, gestattet die Einstellung des Freisetzungsprofils des Wirkstoffes aus der Arzneimittelformulierung so, dass eine gegen Ende der Freisetzung (also nach ca. 8 h) im wesentlichen vollständige Freisetzung möglich ist, ohne die anfängliche Freisetzung wesentlich zu beeinflussen. In anderen Worten wird durch die Verwendung der quellbaren Zwischenschicht ein steilerer Verlauf des Freisetzungsprofils im Vergleich zu einer entsprechenden Formulierung ohne Zwischenschicht erzielt. Dies bedeutet, dass gegen Ende die aus der Arzneimittelformulierung freigesetzte Menge an Wirkstoff substantiell über derjenigen Menge liegt, die aus einer entsprechenden Formulierung ohne Zwischenschicht freigesetzt wird.

Die quellbare Zwischenschicht kann zudem das Freisetzungsprofil dahingehend modulieren, dass die Freisetzung gegenüber derjenigen der entsprechenden Formulierung ohne Zwischenschicht anfänglich retardiert ist bzw. verringert. Damit kann ein noch steileres Freisetzungsprofil erzielt werden.

Vorzugsweise beträgt die Freisetzung nach 8 Stunden, gemessen nach der Drehkörbchenmethode gemäß Ph.Eur.2.9.3-1, mindestens 75 % der Gesamtmenge des in der Formulierung enthaltenden Wirkstoffes, stärker bevorzugt mindestens 85 % der Gesamtmenge und am meisten bevorzugt mindestens 90 % der Gesamtmenge des in der Formulierung enthaltenden Wirkstoffes. Die freigesetzte Menge an Wirkstoff beträgt nach 8 Stunden weiterhin vorzugsweise mindestens 130 % derjenigen Menge, die von der entsprechenden Formulierung ohne Zwischenschicht in derselben Zeit freigesetzt wird, wiederum gemessen nach der Drehkörbchenmethode gemäß Ph.Eur.2.9.3-l. Stärker bevorzugt beträgt diese Menge mindestens 150 % und noch stärker bevorzugt mindestens 180 % der aus der entsprechenden Formulierung ohne Zwischenschicht freigesetzten Menge. Alle Prozentangaben sind hierin in Gewicht/Gewicht.

Weiterhin ist bevorzugt ein Freisetzungsprofil, bei dem die anfängliche Freisetzung nach 1 Stunde, ebenfalls gemessen nach der Drehkörbchenmethode wie oben, zwischen 5 und 35 % (Gewicht/Gewicht) der Gesamtmenge des in der Formulierung enthaltenden Wirkstoffes beträgt. Stärker bevorzugt beträgt diese Menge 15 bis 25 % der Gesamtmenge an Wirkstoff. Bezogen auf diejenige Menge, die von der entsprechenden Formulierung ohne Zwischenschicht freigesetzt wird, beträgt die anfänglich freigesetzte Menge an Wirkstoff nach 1 Stunde, ebenfalls gemessen nach Drehkörbchenmethode, vorzugsweise 70 bis 120 %, noch stärker bevorzugt 90 bis 120 %.

In einer bevorzugten Ausführungsform weist die Arzneimittelformulierung ein Freisetzungsprofil, gemessen nach Drehkörbchenmethode, in Prozent der Gesamtmenge an in der Formulierung enthaltenem Wirkstoff wie folgt auf:

| Zeit (h) | Prozent |
|---|---|
| 1 | 5 - 35 |
| 3 | 45 - 75 |
| 8 | 85 - 100 |

In einer anderen Ausführungsform weist die Arzneimittelformulierung ein Freisetzungsprofil, gemessen nach Drehkörbchenmethode, in Prozent der Gesamtmenge an in der Formulierung enthaltenem Wirkstoff wie folgt auf:

| Zeit (h) | Prozent |
|---|---|
| 1 | 5 - 15 |
| 3 | 25 - 35 |
| 8 | 70 - 80 |
| 12 | 85 - 95 |

Die obigen Freisetzungsprofile und vorteilhaften Ausgestaltungen der Erfindung lassen sich durch Verwendung einer quellbaren Zwischenschicht erzielen, welche ein quellbares Material umfasst. Dieses ist ausgewählt aus der Gruppe, bestehend aus Stärke, Stärkederivaten, Cellulose, Celluloseethern, Carrageenanen, Alginaten, Pektinen, Xanthanen und deren Derivaten, Guar Gummi, Tragant, Polyvinylpyrrolidonen und Mischungen davon. Für die Stärkederivate sind insbesondere vorgelierte Stärke, Natrium-Carboxymethylstärke, Natrium-Stärkeglykolat und ähnliche zu nennen. Für die Celluloseether sind insbesondere Hydroxypropylmethylcellulose (HPMC), Hydroxypropylcellulose (HPC), Hydroxyethylcellulose (HEC), Methylcellulose (MC) und Natriumcarboxymethylcellulose zu nennen. Ganz besonders bevorzugt handelt es sich um Natriumcarboxymethylcellulose, Natriumstärkeglykolat, Celluloseether, hier insbesondere Hydroxypropylmethylcellulose, Natrium-Carboxymethylcellulose und Polyvinylpyrrolidone, insbesondere quervernetzte Polyvinylpyrrolidone.

Die o.g. natürlichen oder synthetischen Polymere sind "quellbar" im Sinne der Erfindung, indem sie unter Volumenzunahme Wasser oder ein anderes Fluid wie Salzlösungen, alkoholische Lösungen, Magensaft etc. einlagern können. Die Volumenzunahme beträgt dabei mindestens das 1,5 fache des Trockenvolumens, stärker bevorzugt mindestens das 5—20 fache. Die Polymere haben üblicherweise ein Molekulargewicht im Bereich von 10.000 bis 2.000.000. Sie können quervernetzt sein. Entsprechende Polymere sind im Handel erhältlich. Geeignet sind bspw. das unter der Bezeichnung Pharmacoat 606 bzw. 615 vertriebene Hydroxypropylmethylcellulose-Produkt, sowie die Produkte, welche unter den Bezeichnungen Klucel EF, Klucel LF, Methocel A, Methocel E, Methocel F, Methocel K, Tylose H, Primojel, Ultramyl, Explotab, Ac-Di-Sol, Kollidon XL, Starch 1500 oder Rhodigel vertrieben werden.

Die quellbare Zwischenschicht kann neben dem quellbaren Material zusätzlich einen oder mehrere Hilfsstoffe enthalten, die vorzugsweise ausgewählt werden unter Bindemitteln wie Povidon, Lösungsmitteln wie Wasser oder Alkoholen und deren Mischungen, pH-Modulatoren, Puffersubstanzen, Weichmachern, , Trennmitteln und Füllstoffen und dergleichen. Die Art und Menge des oder der Hilfsstoffe ist solange frei wählbar, solange hierdurch die quellbaren Eigenschaften der Schicht nicht wesentlich beeinträchtigt werden.

Die quellbare Zwischenschicht wird auf das Substrat der Arzneimittelformulierung in einer Menge aufgetragen, die zur Erzielung des gewünschten Effekts auf das Freisetzungsprofil ausreicht. Die Menge an quellbarer Zwischenschicht, die auf das Substrat der Arzneimittelformulierung aufgetragen wird, ist dabei von der Art des quellbaren Materials sowie der Art und Dicke der retardierenden Überzugsschicht abhängig. Eine Optimierung geeigneter Mengen liegt im Können des Fachmanns. Üblicherweise wird die quellbare Zwischenschicht in einer Menge von mindestens 0,01 mg/cm$^2$ auf das Substrat der Arzneimittelformulierung aufgetragen, stärker bevorzugt in einer Menge von 0,01 bis 10 mg/cm$^2$. Noch stärker bevorzugt ist diese Menge in einem Bereich on 0,1 bis 2 mg/cm$^2$, am meisten bevorzugt in einem Bereich von 0,3 bis 1,7 mg/cm$^2$.

Die quellbare Zwischenschicht kann in Form einer oder mehrerer quellbarer Zwischenschichten vorliegen, wobei sich letztere bspw. hinsichtlich des quellbaren Materials oder lediglich der Polymerlänge oder der Art und des Vorhandenseins von Zusatzstoffen unterscheiden können.

Die retardierende Überzugsschicht wird üblicherweise ein Retardpolymer umfassen. Hierzu kann jedes im Stand der Technik bekannte, die Freisetzung verzögernde Retardpolymer verwendet werden. Geeignete Beispiele sind Ethylcellulose, unlösliche Poly(meth)acrylate und/oder Polyvinylacetate. Entsprechende Polymere und deren Lösungen sind auch in Arzneimittelqualität im Handel erhältlich. Bspw. zu nennen sind die Polyacrylate wie Eudragit®NE, Eudragit®RS und Eudragit®RL sowie das Polyvinylacetat Kollicoat SR30D.

Die retardierende Überzugsschicht kann neben dem Retardpolymer zusätzlich ein oder mehrere Hilfsstoffe umfassen, ausgewählt aus der Gruppe, bestehend aus Bindemitteln, Lösungsmitteln wie Wasser, Alkohole und deren Mischungen, pH-Regulatoren, Puffersubstanzen, Weichmachern, Porenbildnern, Sprengmitteln, Retardierungsmitteln, weiteren Filmbildnern, Geschmacksstoffen, Farbstoffen, Pigmenten, Gleitmitteln, weiteren Modifikatoren der Freisetzung wie Gummen, Stärken, Oligosacchariden und ähnlichem, aber auch Trennmittel und Füllstoffen und dergleichen. Bezüglich der verwendbaren Retardpolymere und Hilfsstoffe wird zudem auf die EP-A-1 243 269 und die EP-A-1 419 766 Bezug genommen. Die Art und Menge der Hilfsstoffe ist solange frei wählbar solange die retardierenden Eigenschaften der Überzugsschicht nicht wesentlich beeinträchtigt werden.

Der Retardfilm wird üblicherweise in einer Filmdicke von 0,1 bis 20 mg/cm$^2$, vorzugsweise 0,5 bis 10 mg/ cm$^2$ und noch stärker bevorzugt 1 bis 5,5 mg/ cm$^2$ aufgetragen. Die Wahl der Dicke der retardierenden,Überzugsschicht hängt für den Fachmann ersichtlich von der Art des gewählten Retardpolymeren, der gewünschten Verzögerung der Freisetzung sowie der Art und Menge der verwendeten Hilfsstoffe ab. Die Einstellung der entsprechenden Eigenschaften dieser Überzugsschicht ist aus den oben genannten Dokumenten des Standes der Technik bekannt. Ihre Optimierung liegt im Können des Fachmanns. Die Überzugsschicht kann in mehrere Schichten aufgeteilt sein.

Die erfindungsgemäße feste, verzögerte freisetzende Arzneimittelformulierung zur peroralen Verabreichung enthält ein den oder die Wirkstoff(e) enthaltendes Substrat oder Kern, die wie oben beschriebe(n) Zwischenschicht(en) und eine retardierende Überzugsschicht. Weitere Schichten, wie geschmacks- oder optikverbessernde Überzüge, interne Sperrschichten können vorhanden sein. Die Formulierung ist typischerweise eine Formulierung, ausgewählt aus der Gruppe, bestehend aus Tabletten, Kapseln, Pellets, Mikrotabletten, Nanopellets, Nanotabletten, Granulat, Kristallen sowie in Kapseln oder Sachets abgefüllten Pellets, Mikrotabletten, Nanopellets, Nanotabletten, Granulat und Kristallen. Bevorzugt handelt es sich bei der Arzneimittelformulierung um Tabletten, Pellets, Mikrotabletten oder Kapseln noch stärker bevorzugt um Pellets oder Mikrotabletten. Pellets oder Mikrotabletten weisen vorzugsweise einen Durchmesser (ohne Beschichtung) von 0,2 bis 2,0 mm (vorzugsweise 0,7 bis 1,2 mm) auf, können jedoch Durchmesser von bis zu 5 mm aufweisen. Kleinteilige Formen wie (Nano)pellets, Mikro- oder Nanotabletten, Kristalle, Pulver oder Granulat können zu zerfallenden Tabletten verpresst sein.

Die erfindungsgemäße Arzneimittelformulierung weist ein wirkstoffhaltiges Substrat auf. Dieses Substrat kann den Wirkstoff als solchen oder in Kombination mit üblichen Hilfsstoffen enthalten. Der Wirkstoff kann bspw. mit Hilfsstoffen durch Anwendung von Extrusions-Spheronisations- oder Tropfverfahren zu Pellets verarbeitet werden. Ebenso kann der Wirkstoff zu Tabletten oder Mikrotabletten verpresst werden oder auf entsprechende Tabletten- und Pelletkerne wie Non-pareille-Pellets aufgesprüht werden. Alternativ kann es sich bei dem Substrat bereits um mit Wirkstoff z.B. in Form von Pellets, Pulver, Mikrotabletten, Nanopellets, Nanotabletten, Granulat, Kristallen oder ähnlichem gefüllt Kapseln handeln, die ihrerseits mit der Zwischenschicht und verzögert freisetzenden Schicht gemäß der vorliegenden Erfindung versehen werden.

Der erfindungsgemäße Effekt der Steuerung des Freisetzungsprofils für verzögert freisetzende feste perorale Arzneiformen ist prinzipiell für alle peroral verabreichbaren Wirkstoffe anwendbar. Bevorzugt ist der Wirkstoff ausgewählt aus der Gruppe, bestehend aus Antihistaminen (z.B. Dimenhydrinat, Diphenhydramin, Chlorpheniramin, Cetirizin, Loratadin, und Dexchlorpheniraminmaleat), Analgetika (z.B. Hydromorphon, Acetysalicylsäure, Codein, Morphin, Dihydromorphon, Oxycodon, etc.), nicht-steroidalen Entzündungshemmern (z.B. Naproxen, Diclofenac, Indomethacin, Ibuprofen, Sulindac), Antiemetika (z.B. Metoclopramid), Antiepileptika (z.B. Phenytoin, Meprobamat und Nitrezepam), Vasodilatatoren (z.B. Nifedipin, Papaverin, Diltiazem und Nicardirin), anti-tussiven Mitteln und Expectorantien (z.B. Codeinphosphat), Antiasthmatika (z.B. Theophyllin), Spasmolytika (z.B. Atropin, Scopolamin), Mitteln gegen Diabetes (z.B. Insulin und orale Antidiabetika), Diuretika (z.B. Ethacrynsäure, Bendrofluazid), Mitteln gegen niedrigen Blutdruck (z.B. Propranolol, Clonidin), Mitteln gegen Bluthochdruck (e.g. Clonidin, Methyldopa), Bronchodilatatoren (z.B. Albuterol), Steroiden (z.B. Testosteron, Androgen, Estradiol und seinen Derviaten, Hydrocortison, Triamcinolon, Prednison), Antibiotika (z.B. Tetracyclin), antihämorrhoidalen Mitteln, Hypnotika, psychotropen Mitteln, Antidepressiva (z.B. Amitriptylin, Venlafaxin), Mitteln gegen Durchfall, Mucolytika, Sedativa, Decongestanzien, Laxanzien, Vitaminen und Stimulanzien (eingeschlossen Appetitzügler wie Phenylpropanolamin). Stärker bevorzugt handelt es sich um ein Analgetikum, ein Antidepressivum oder nicht steroidale Entzündungshemmer, am meisten bevorzugt um Hydromorphon oder Amitriptylin.

Die vorliegende Erfindung trifft auch verzögert freisetzende, feste Arzneimittelformulierungen zur peroralen Anwendung, die durch Verwendung der quellbaren Zwischenschicht gekennzeichnet sind. Die Arzneimittelformulierungen sind wie oben und in den Ansprüchen beschrieben.

Am meisten bevorzugt handelt es sich um Arzneimittelformulierungen in Form von Pellets mit einem Durchmesser von 0,2 bis 2 mm, welche Hydromorphon oder Amitriptylin oder arzneimittelverträgliche Salze davon als Wirkstoff enthalten. Die Zwischenschicht wird erfindungsgemäß bevorzugt aus Hydroxypropylmethylcellulose oder Natriumcarboxymethylcellulose ggf. mit Hilfsstoffen bestehen, wobei Retardfilme aus Polyvinylacetat und/oder Ethylcellulose verwendet werden.

Die folgenden Beispiele sollen die Erfindung lediglich veranschaulichen, diese jedoch nicht einschränken.

**Beispiel 1:**

Herstellung des Pelletkerns mit Hilfe eines Extrusionsverfahrens. Zwischenschicht und Retardfilm werden in der Wirbelschicht aufgebracht. Die eingesetzten Pellets haben einen Durchmesser von 710 -1200 μm.

**Zusammensetzung [mg/DF]**

| | Inhaltsstoffe | [mg/DF] | [mg/DF] | Filmdicke mg/cm$^2$ | Funktion |
|---|---|---|---|---|---|
| | | A | B | | |
| **Pelletkern** | Hydromorphon- HCl | 16,0 | 16,0 | | Wirkstoff |
| | Avicel PH 101 | 163,0 | 163,0 | | Extrusionshilfsstoff |
| | Methocel E5 | 1,0 | 1,0 | | Bindemittel |
| | | | | | |
| **Zwischenschicht** | Pharmacoat 606 | 5,4 | --- | 0,8 | Filmbildner |
| | | | | | |
| **Retardfilm** | Kollicoat SR30D | 25,2 | 25,2 | | Filmbildner |
| | Triethylcitrat | 2,52 | 2,52 | 4,0 | Weichmacher |
| | Syloid 244 FP | 3,03 | 3,03 | | Trennmittel |
| | Kollidon 30 | 5,05 | 5,05 | | Filmbildner |

**In-Vitro Freisetzungsprofil**

Paddle Apparatur gemäß Ph.Eur. 2.9.3-1, 100 rpm, Phosphatpuffer pH 6.4 gemäß USP, 900 ml, n=6

| Zeit (h) | Mittelwert [%] A | Mittelwert [%] B |
|---|---|---|
| 1 | 31,6 | 25,2 |
| 2 | 57,5 | 43,4 |
| 3 | 71,8 | 53,2 |
| 4 | 82,0 | 58,5 |
| 5 | 86,5 | 62,5 |
| 6 | 90,2 | 65,4 |
| 7 | 93,4 | 68,2 |
| 8 | 95,3 | 70,4 |

**Beispiel 2:**

Herstellung des Pelletkerns mit Hilfe eines Extrusionsverfahrens. Zwischenschicht und Retardfilm werden in der Wirbelschicht aufgebracht. Die eingesetzten Pellets haben einen Durchmesser von 710-1200 μm.

**Zusammensetzung [mg/DF]**

| | Inhaltsstoffe | [mg/DF] A | [mg/DF] B | Filmdicke mg/cm$^2$ | Funktion |
|---|---|---|---|---|---|
| **Pelletkern** | Hydromorphon-HCl | 16,0 | 16,0 | | Wirkstoff |
| | Avicel PH 101 | 163,0 | 163,0 | | Extrusionshilfsstoff |
| | Methocel E5 | 1,0 | B | | Bindemittel |
| | | | | | |
| **Zwischenschicht** | Pharmacoat 606 | 3,6 | --- | 0,5 | Filmbildner |
| | | | | | |
| **Retardfilm** | Ethylcellulose | 4,5 | 4,5 | | Filmbildner |
| | Dibutylsebacat | 0,96 | 0,96 | 1,0 | Weichmacher |
| | Ölsäure | 0,54 | 0,54 | | Netzmittel |
| | Siliciumdioxid | 0,49 | 0,49 | | Trennmittel |

**In-Vitro Freisetzungsprofil**

Paddle Apparatur gemäß Ph.Eur. 2.9.3-1, 100 rpm, Phosphatpuffer pH 6.4 gemäß USP, 900 ml, n=6

| Zeit (h) | Mittelwert [%] A | Mittelwert [%] B |
|---|---|---|
| 1 | 19,0 | 11,6 |
| 3 | 67,2 | 29,2 |
| 8 | 100,3 | 65,7 |

**Beispiel 3:**

Herstellung des Pelletkerns mit Hilfe eines Extrusionsverfahrens. Zwischenschicht und Retardfilm werden in der Wirbelschicht aufgebracht. Die eingesetzten Pellets haben einen Durchmesser von 710-1200 μm.

**Zusammensetzung [mg/DF]**

|  | Inhaltsstoffe | [mg/DF] | [mg/DF] | Filmdicke mg/cm$^2$ | Funktion |
|---|---|---|---|---|---|
|  |  | A | B |  |  |
| **Pelletkern** | Hydromorphon-HCl | 16,0 | 16,0 |  | Wirkstoff |
|  | Avicel PH 101 | 163,0 | 163,0 |  | Extrusionshilfsstoff |
|  | Methocel E5 | 1,0 | 1,0 |  | Bindemittel |
|  |  |  |  |  |  |
| **Zwischenschicht** | Pharmacoat 606 | 1,8 | --- | 0,3 | Filmbildner |
|  |  |  |  |  |  |
| **Retardfilm** | Ethylcellulose | 5,4 | 5,4 |  | Filmbildner |
|  | Dibutylsebacat | 1,15 | 1,15 | 1,0 | Weichmacher |
|  | Ölsäure | 0,65 | 0,65 |  | Netzmittel |
|  | Siliciumdioxid | 0,59 | 0,59 |  | Trennmittel |

**In-Vitro Freisetzungsprofil**

Paddle Apparatur gemäß Ph.Eur. 2.9.3-1, 100 rpm, Phosphatpuffer pH 6.4 gemäß USP, 900 ml, n=6

| Zeit (h) | Mittelwert [%] A | Mittelwert [%] B |
|---|---|---|
| 1 | 9,6 | 4,3 |
| 3 | 49,7 | 9,2 |
| 8 | 88,6 | 31,1 |

**Beispiel 4:**

Herstellung des Pelletkerns mit Hilfe eines Extrusionsverfahrens. Zwischenschicht und Retardfilm werden in der Wirbelschicht aufgebracht. Die eingesetzten Pellets haben einen Durchmesser von 710 -1200 μm.

**Zusammensetzung [mg/DF]**

|  | Inhaltsstoffe | [mg/DF] | [mg/DF] | Filmdicke mg/cm$^2$ | Funktion |
|---|---|---|---|---|---|
|  |  | A | B |  |  |
| **Pelletkern** | Amitriptylin-HCl | 75,0 | 75,0 |  | Wirkstoff |
|  | Ascorbinsäure | 15,0 | 15,0 |  | Stabilisator |
|  | Avicel PH 101 | 138,8 | 138,8 |  | Extrusionshilfsstoff |
|  | Maisstärke | 16,25 | 16,15 |  | Füllstoff |

(fortgesetzt)

| | Inhaltsstoffe | [mg/DF] | [mg/DF] | Filmdicke mg/cm$^2$ | Funktion |
|---|---|---|---|---|---|
| | | A | B | | |
| | | | | | |
| **Zwischenschicht** | Povidon 25 | 2,45 | --- | | Bindemittel |
| | Na-Carboxy-methylstärke | 12,15 | --- | 1,7 | Quellmittel |
| | | | | | |
| **Retardfilm** | Kollicoat SR (Polymer) | 34,3 | 34,3 | | Filmbildner |
| | Triethylcitrat | 3,43 | 3,43 | 4,5 | Weichmacher |
| | Povidon 30 | 5,48 | 5,48 | | Filmbildner |
| | Syloid | 4,11 | 4,11 | | Trennmittel |

**In-Vitro Freisetzungsprofil**

Paddle Apparatur gemäß Ph.Eur. 2.9.3-1, 100 rpm, Phosphatpuffer pH 6.8 gemäß USP, 900 ml, n=6

| Zeit (h) | Mittelwert [%] A | Mittelwert [%] B |
|---|---|---|
| 1 | 6,3 | 7,8 |
| 2 | 14,6 | 13,4 |
| 3 | 27,5 | 20,8 |
| 4 | 40,6 | 27,5 |
| 5 | 53,3 | 33,6 |
| 6 | 63,9 | 39,3 |
| 8 | 75,6 | 50,9 |
| 10 | 82,0 | 61,5 |
| 12 | 86,1 | 68,6 |

**Beispiel 5:**

Herstellung des Pelletkerns mit Hilfe eines Extrusionsverfahrens. Zwischenschicht und Retardfilm werden in der Wirbelschicht aufgebracht. Die eingesetzten Pellets haben einen Durchmesser von 710 -1200 μm.

**Zusammensetzung [mg/DF]**

| | Inhaltsstoffe | [mg/DF] | [mg/DF] | Filmdicke mg/cm$^2$ | Funktion |
|---|---|---|---|---|---|
| | | A | B | | |
| **Pelletkern** | Amitriptylin-HCl | 75,0 | 75,0 | | Wirkstoff |
| | Ascorbinsäure | 15,0 | 15,0 | | Stabilisator |
| | Avicel PH 101 | 138,8 | 138,8 | | Extrusionshilfsstoff |
| | Maisstärke | 16,25 | 16,15 | | Füllstoff |

(fortgesetzt)

| | Inhaltsstoffe | [mg/DF] | [mg/DF] | Filmdicke mg/cm² | Funktion |
|---|---|---|---|---|---|
| | | A | B | | |
| | | | | | |
| **Zwischenschicht** | Povidon 25 | 2,45 | --- | | Bindemittel |
| | Na-Carboxymethylstärke | 12,15 | --- | 1,7 | Quellmittel |
| | | | | | |
| **Retardfilm** | Kollicoat SR (Polymer) | 39,2 | 39,2 | | Filmbildner |
| | Triethylcitrat | 3,92 | 3,92 | 4,5 | Weichmacher |
| | Povidon 30 | 6,27 | 6,27 | | Filmbildner |
| | Syloid | 4,70 | 4,70 | | Trennmittel |

**In-Vitro Freisetzungsprofil**

Paddle Apparatur gemäß Ph.Eur. 2.9.3-1, 100 rpm, Phosphatpuffer pH 6.8 gemäß USP, 900 ml, n=6

| Zeit (ha) | Mittelwert [%] A | Mittelwert [%] B |
|---|---|---|
| 1 | 9,3 | 4,5 |
| 2 | 19,1 | 6,8 |
| 3 | 31,5 | 12,1 |
| 4 | 43,5 | 18,2 |
| 5 | 53,8 | 24,3 |
| 6 | 62,7 | 30,5 |
| 8 | 75,2 | 41,6 |
| 10 | 82,4 | 52,4 |
| 12 | 86,5 | 59,0 |

Aus den oben gegebenen Beispielen ist ersichtlich, dass sich durch Verwendung einer quellbaren Zwischenschicht gemäß der vorliegenden Erfindung eine deutliche Erhöhung der Freisetzung gegen Ende des Freisetzungsprofils erzielen lässt. Damit lässt sich die Abflachung des Freisetzungsprofils gegen Ende aufheben und somit eine im Wesentlichen vollständige Freisetzung des Wirkstoffes erreichen. Diese vorliegenden Beispiele sollen die Erfindung lediglich veranschaulichen, diese jedoch nicht einschränken.

**Patentansprüche**

1. Verwendung einer quellbaren Zwischenschicht, umfassend ein quellbares Material, ausgewählt aus der Gruppe, bestehend aus Stärke, Stärkederivaten wie vorgelierte Stärke, Na-Carboxyethylstärke, Na-Stärkeglykolat, Cellulose, Celluloseethern wie HPMC, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, in einer verzögert freisetzenden, festen Arzneimittelformulierung zur peroralen Anwendung, welche Formulierung ein einen Wirkstoff enthaltendes Substrat, die quellbare Zwischenschicht und eine retardierende Überzugsschicht aufweist, zur Einstellung eines Freisetzungsprofils des Wirkstoffes, welches gegen Ende eine im Wesentlichen vollständige Freisetzung gestattet.

2. Verwendung nach Anspruch 1, worin das Freisetzungsprofil zudem gegenüber demjenigen der entsprechenden Formulierung ohne Zwischenschicht anfänglich retardiert ist.

**3.** Verwendung nach Anspruch 1 oder 2, worin die Freisetzung nach 8 h, gemessen nach der Drehkörbchenmethode gemäß Ph. Eur 2.9.3-1, mindestens 75 % der Gesamtmenge des in der Formulierung enthaltenden Wirkstoffes beträgt.

**4.** Verwendung nach Anspruch 1 oder 2, worin die freigesetzte Menge an Wirkstoff nach 8 h, gemessen nach der Drehkörbchenmethode gemäß Ph. Eur 2.9.3-1, mindestens 130 % derjenigen der entsprechenden Formulierung ohne Zwischenschicht beträgt.

**5.** Verwendung nach einem der Ansprüche 2 bis 4, worin die Freisetzung nach 1 h, gemessen nach der Drehkörbchenmethode gemäß Ph. Eur 2.9.3-1, zwischen 5 und 35 % der Gesamtmenge des in der Formulierung enthaltenden Wirkstoffes beträgt.

**6.** Verwendung nach einem der Ansprüche 2 bis 4, worin die freigesetzte Menge an Wirkstoff nach 1 h, gemessen nach der Drehkörbchenmethode gemäß Ph. Eur 2.9.3-1, zwischen 70 und 120 % derjenigen Menge beträgt, die von der entsprechenden Formulierung ohne Zwischenschicht freigesetzt wird.

**7.** Verwendung nach einem der vorstehenden Ansprüche, worin die Arzneimittelformulierung das folgende Freisetzungsprofil, gemessen nach der Drehkörbchenmethode gemäß Ph. Eur 2.9.3-1, in Prozent der Gesamtmenge an in der Formulierung enthaltenem Wirkstoff aufweist:

| Zeit (h) | Prozent |
|----------|----------|
| 1 | 5 - 35 |
| 3 | 45 - 75 |
| 8 | 85 - 100 |

**8.** Verwendung nach einem der vorstehenden Ansprüche, worin die Arzneimittelformulierung das folgende Freisetzungsprofil, gemessen nach der Drehkörbchenmethode gemäß Ph. Eur 2.9.3-1, in Prozent der Gesamtmenge an in der Formulierung enthaltenem Wirkstoff aufweist:

| Zeit (h) | Prozent |
|----------|----------|
| 1 | 5 - 15 |
| 3 | 25 - 35 |
| 8 | 70 - 80 |
| 12 | 85 - 95 |

**9.** Verwendung nach einem der vorstehenden Ansprüche, worin es sich bei der per-oralen Arzneimittelformulierung um eine Formulierung handelt, ausgewählt aus der Gruppe, bestehend aus Tabletten, Kapseln, Pellets, Mikrotabletten, Nanopellets, Nanotabletten, Granulat, Kristallen sowie in Kapseln oder Sachets abgefüllten oder zu Tabletten verpressten Pellets, Mikrotabletten, Nanopellets, Nanotabletten, Granulat und Kristallen.

**10.** Verwendung nach Anspruch 9, worin es sich bei der Arzneimittelformulierung um Tabletten, Pellets, Mikrotabletten oder Kapseln handelt, vorzugsweise um Pellets oder Mikrotabletten mit einem Durchmesser (ohne Beschichtung) von 0,2 bis 2,0 mm.

**11.** Verwendung nach einem der vorstehenden Ansprüche, worin die quellbare Zwischenschicht in einer Menge von mindestens 0,01 mg/cm$^2$ auf das Substrat der Arzneimittelformulierung aufgetragen wird.

**12.** Verwendung nach Anspruch 11, worin die Zwischenschicht in einer Menge von 0,01 bis 10 mg/cm$^2$ aufgetragen wird.

**13.** Verwendung nach einem der vorstehenden Ansprüche, worin das quellbare Material, ausgewählt ist unter Na-Carboxyethylstärke, Na-Stärkeglykolat, Celluloseethern, Na-Carboxymethylcellulose, Polyvinylpyrrolidonen und Mischungen davon.

**14.** Verwendung nach Anspruch 13, worin die Celluloseether ausgewählt sind unter Hydroxypropylcellulose (HPMC), Hydroxypropylcellulose (HPC), Hydroxyethylcellulose (HEC) und Methylcellulose (MC).

**15.** Verwendung nach einem der vorstehenden Ansprüche, worin die Zwischenschicht zusätzlich einen oder mehrere Hilfsstoffe enthält, ausgewählt unter Bindemitteln, Lösungsmitteln, pH-Regulatoren, Puffersubstanzen, Weichmachern, Trennmitteln und Füllstoffen.

**16.** Verwendung nach einem der vorstehenden Ansprüche, worin die retardierende Überzugsschicht ein Retardpolymer umfasst.

**17.** Verwendung nach Anspruch 16, worin das Retardpolymer ausgewäht ist aus der Gruppe, bestehend aus Celluloseethern wie Ethylcellulose, unlöslichen Polyacrylaten wie Eudragit®NE, Eudragit®RS und Eudragit®RL, Polyvinylacetaten wie Kollicoat SR30D.

**18.** Verwendung nach Anspruch 16 oder 17, worin die retardierende Überzugsschicht zusätzlich einen oder mehrere Hilfsstoffe umfasst, ausgewählt aus der Gruppe, bestehend aus Bindemitteln, Lösungsmitteln, pH-Regulatoren, Puffersubstanzen, Weichmachern, Porenbildnern, Filmbildner, Sprengmitteln, Retardierungsmitteln, Geschmacksstoffen, Farbstoffen, Pigmenten, Gleitmitteln, Freisetzungsmodifikatoren, Trennmitteln und Füllstoffen.

**19.** Verwendung nach einem der vorstehenden Ansprüche, worin der Wirkstoff ausgewählt ist aus der Gruppe, bestehend aus Antihistaminen, Analgetika, Nicht-steroidalen Entzündungshemmern, Antiemetika, Antiepileptika, Vasodilatatoren, anti-tussiven Mitteln und Expectorantien, Antiasthmatika, Spasmolytika, Mitteln gegen Diabetes, Diuretika. Mitteln gegen niedrigen Blutdruck, Mitteln gegen Bluthochdruck, Bronchodilatatoren, Steroiden, Antibiotika, antihämorrhoidalen Mitteln, Hypnotika, psychotropen Mitteln, Antidepressiva, Mitteln gegen Durchfall, Mucolytika, Sedativa, Decongestanzien, Laxanzien, Vitaminen und Stimulanzien.

**20.** Verwendung nach einem der vorstehenden Ansprüche, worin der Wirkstoff Hydromorphon oder Amitriptylin oder ein pharmazeutisch annehmbares Salz davon ist.

**21.** Verzögert freisetzende, feste Arzneimittelformulierung zur peroralen Anwendung, welche Formulierung ein einen Wirkstoff enthaltendes Substrat, eine quellbare Zwischenschicht und eine retardierende Überzugsschicht aufweist, worin die quellbare Zwischenschicht ein quellbares Material umfasst, ausgewählt aus der Gruppe, bestehend aus Stärke, Stärkederivaten wie pregelatinisierter Stärke, Na-Carboxyethylstärke, Na-Stärkeglykolat, Cellulose, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, und in einer zur Einstellung eines Freisetzungsprofils des Wirkstoffes, welches gegen Ende eine im Wesentlichen vollständige Freisetzung gestattet, wirksamen Menge vorhanden ist.

**22.** Verzögert freisetzende, feste Arzneimittelformulierung nach Anspruch 25, worin das Freisetzungsprofil wie in einem der Ansprüche 2 bis 8 definiert ist.

**Geänderte Patentansprüche gemäss Regel 86(2) EPÜ.**

**1.** Verwendung einer quellbaren Zwischenschicht, umfassend ein quellbares Material, ausgewählt aus der Gruppe, bestehend aus Stärke, Stärkederivaten wie vorgelierte Stärke, Na-Carboxyethylstärke, Na-Stärkeglykolat, Cellulose, Celluloseethern wie HPMC, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, in einer verlängert kontinuierlich freisetzenden, festen Arzneimittelformulierung zur peroralen Anwendung, welche Formulierung ein einen Wirkstoff enthaltendes Substrat, die quellbare Zwischenschicht und eine retardierende Überzugsschicht aufweist, zur Einstellung eines Freisetzungsprofils des Wirkstoffes, welches gegen Ende eine im Wesentlichen vollständige Freisetzung gestattet.

**2.** Verwendung nach Anspruch 1, worin das Freisetzungsprofil zudem gegenüber demjenigen der entsprechenden Formulierung ohne Zwischenschicht anfänglich retardiert ist.

**3.** Verwendung nach Anspruch 1 oder 2, worin die Freisetzung nach 8 h, gemessen nach der Drehkörbchenmethode gemäß Ph. Eur 2.9.3-1, mindestens 75 % der Gesamtmenge des in der Formulierung enthaltenden Wirkstoffes beträgt.

**4.** Verwendung nach Anspruch 1 oder 2, worin die freigesetzte Menge an Wirkstoff nach 8 h, gemessen nach der Drehkörbchenmethode gemäß Ph. Eur 2.9.3-1, mindestens 130 % derjenigen der entsprechenden Formulierung ohne Zwischenschicht beträgt.

**5.** Verwendung nach einem der Ansprüche 2 bis 4, worin die Freisetzung nach 1 h, gemessen nach der Drehkörbchenmethode gemäß Ph. Eur 2.9.3-1, zwischen 5 und 35 % der Gesamtmenge des in der Formulierung enthaltenden Wirkstoffes beträgt.

**6.** Verwendung nach einem der Ansprüche 2 bis 4, worin die freigesetzte Menge an Wirkstoff nach 1 h, gemessen nach der Drehkörbchenmethode gemäß Ph. Eur 2.9.3-1, zwischen 70 und 120 % derjenigen Menge beträgt, die von der entsprechenden Formulierung ohne Zwischenschicht freigesetzt wird.

**7.** Verwendung nach einem der vorstehenden Ansprüche, worin die Arzneimittelformulierung das folgende Freisetzungsprofil, gemessen nach der Drehkörbchenmethode gemäß Ph. Eur 2.9.3-1, in Prozent der Gesamtmenge an in der Formulierung enthaltenem Wirkstoff aufweist:

| Zeit (h) | Prozent |
|----------|---------|
| 1 | 5 - 35 |
| 3 | 45 - 75 |
| 8 | 85 - 100 |

**8.** Verwendung nach einem der vorstehenden Ansprüche, worin die Arzneimittelformulierung das folgende Freisetzungsprofil, gemessen nach der Drehkörbchenmethode gemäß Ph. Eur 2.9.3-1, in Prozent der Gesamtmenge an in der Formulierung enthaltenem Wirkstoff aufweist:

| Zeit (h) | Prozent |
|----------|---------|
| 1 | 5 - 15 |
| 3 | 25 - 35 |
| 8 | 70 - 80 |
| 12 | 85 - 95 |

**9.** Verwendung nach einem der vorstehenden Ansprüche, worin es sich bei der per-oralen Arzneimittelformulierung um eine Formulierung handelt, ausgewählt aus der Gruppe, bestehend aus Tabletten, Kapseln, Pellets, Mikrotabletten, Nanopellets, Nanotabletten, Granulat, Kristallen sowie in Kapseln oder Sachets abgefüllten oder zu Tabletten verpressten Pellets, Mikrotabletten, Nanopellets, Nanotabletten, Granulat und Kristallen.

**10.** Verwendung nach Anspruch 9, worin es sich bei der Arzneimittelformulierung um Tabletten, Pellets, Mikrotabletten oder Kapseln handelt, vorzugsweise um Pellets oder Mikrotabletten mit einem Durchmesser (ohne Beschichtung) von 0,2 bis 2,0 mm.

**11.** Verwendung nach einem der vorstehenden Ansprüche, worin die quellbare Zwischenschicht in einer Menge von mindestens 0,01 mg/cm$^2$ auf das Substrat der Arzneimittelformulierung aufgetragen wird.

**12.** Verwendung nach Anspruch 11, worin die Zwischenschicht in einer Menge von 0,01 bis 10 mg/cm$^2$ aufgetragen wird.

**13.** Verwendung nach einem der vorstehenden Ansprüche, worin das quellbare Material, ausgewählt ist unter Na-Carboxyethylstärke, Na-Stärkeglykolat, Celluloseethern, Na-Carboxymethylcellulose, Polyvinylpyrrolidonen und Mischungen davon.

**14.** Verwendung nach Anspruch 13, worin die Celluloseether ausgewählt sind unter Hydroxypropylcellulose (HPMC), Hydroxypropylcellulose (HPC), Hydroxyethylcellulose (HEC) und Methylcellulose (MC).

**15.** Verwendung nach einem der vorstehenden Ansprüche, worin die Zwischenschicht zusätzlich einen oder mehrere Hilfsstoffe enthält, ausgewählt unter Bindemitteln, Lösungsmitteln, pH-Regulatoren, Puffersubstanzen, Weichmachern, Trennmitteln und Füllstoffen.

**16.** Verwendung nach einem der vorstehenden Ansprüche, worin die retardierende Überzugsschicht ein Retardpolymer umfasst.

**17.** Verwendung nach Anspruch 16, worin das Retardpolymer ausgewäht ist aus der Gruppe, bestehend aus Celluloseethern wie Ethylcellulose, unlöslichen Polyacrylaten wie Eudragit®NE, Eudragit®RS und Eudragit®RL, Polyvinylacetaten wie Kollicoat SR30D.

**18.** Verwendung nach Anspruch 16 oder 17, worin die retardierende Überzugsschicht zusätzlich einen oder mehrere Hilfsstoffe umfasst, ausgewählt aus der Gruppe, bestehend aus Bindemitteln, Lösungsmitteln, pH-Regulatoren, Puffersubstanzen, Weichmachern, Porenbildnem, Filmbildner, Sprengmitteln, Retardierungsmitteln, Geschmacksstoffen, Farbstoffen, Pigmenten, Gleitmitteln, Freisetzungsmodifikatoren, Trennmitteln und Füllstoffen.

**19.** Verwendung nach einem der vorstehenden Ansprüche, worin der Wirkstoff ausgewählt ist aus der Gruppe, bestehend aus Antihistaminen, Analgetika, Nicht-steroidalen Entzündungshemmem, Antiemetika, Antiepileptika, Vasodilatatoren, anti-tussiven Mitteln und Expectorantien, Antiasthmatika, Spasmolytika, Mitteln gegen Diabetes, Diuretika. Mitteln gegen niedrigen Blutdruck, Mitteln gegen Bluthochdruck, Bronchodilatatoren, Steroiden, Antibiotika, antihämorrhoidalen Mitteln, Hypnotika, psychotropen Mitteln, Antidepressiva, Mitteln gegen Durchfall, Mucolytika, Sedativa, Decongestanzien, Laxanzien, Vitaminen und Stimulanzien.

**20.** Verwendung nach einem der vorstehenden Ansprüche, worin der Wirkstoff Hydromorphon oder Amitriptylin oder ein pharmazeutisch annehmbares Salz davon ist.

**21.** Verlängert kontinuierlich freisetzende, feste Arzneimittelformulierung zur peroralen Anwendung, welche Formulierung ein einen Wirkstoff enthaltendes Substrat, eine quellbare Zwischenschicht und eine retardierende Überzugsschicht aufweist, worin die quellbare Zwischenschicht ein quellbares Material umfasst, ausgewählt aus der Gruppe, bestehend aus Stärke, Stärkederivaten wie pregelatinisierter Stärke, Na-Carboxyethylstärke, Na-Stärkeglykolat, Cellulose, HPC, HEC, MC, Na-Carboxymethylcellulose, Carrageenanen, Alginaten, Pektinen, Xanthanen und deren Derivaten, Guar Gummi, Tragant, Polyvinylpyrrolidonen und Mischungen davon, und in einer zur Einstellung eines Freisetzungsprofils des Wirkstoffes, welches gegen Ende eine im Wesentlichen vollständige Freisetzung gestattet, wirksamen Menge vorhanden ist.

**22.** Verzögert freisetzende, feste Arzneimittelformulierung nach Anspruch 21, worin das Freisetzungsprofil wie in einem der Ansprüche 2 bis 8 definiert ist.

EP 1 754 471 A1

Europäisches
Patentamt

**EUROPÄISCHER RECHERCHENBERICHT**

Nummer der Anmeldung

EP 05 01 7958

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 610 323 B1 (LUNDBERG PER JOHAN ET AL) 26. August 2003 (2003-08-26) * Spalte 9, Zeile 59 - Spalte 11, Zeile 41 * * Beispiel 1 * ----- | 1,9-19 | A61K9/50 A61K9/20 |
| X | US 5 766 623 A (AYRES ET AL) 16. Juni 1998 (1998-06-16) * Spalte 3, Zeilen 38-54 * * Spalte 5, Zeilen 12-51 * ----- | 1,9, 13-19 | |
| D,X | EP 1 419 766 A (EURO-CELTIQUE S.A) 19. Mai 2004 (2004-05-19) * Zusammenfassung * * Absätze [0016], [0027], [0028] * * Absätze [0044] - [0051] * * Beispiele * ----- | 1-22 | |
| D,X | EP 1 243 269 A (EURO-CELTIQUE S.A) 25. September 2002 (2002-09-25) * Absätze [0024], [0025], [0030], [0031] * * Absätze [0036], [0037], [0045] * * Beispiele * ----- | 1-22 | |
| X | EP 0 572 942 A (POLI INDUSTRIA CHIMICA S.P.A; MONSANTO ITALIANA S.P.A; POLICHEM S.A) 8. Dezember 1993 (1993-12-08) * Zusammenfassung * * Seite 2, Zeile 47 - Seite 3, Zeile 35 * * Beispiele * * Ansprüche * ----- | 1-22 | |
| Y | US 5 891 474 A (BUSETTI ET AL) 6. April 1999 (1999-04-06) * Zusammenfassung * * Spalte 5, Zeilen 27-64 * * Beispiele * ----- | 1-22 | |

RECHERCHIERTE SACHGEBIETE (IPC)

A61K

-/--

Der vorliegende Recherchenbericht wurde für alle Patentansprüche erstellt

| Recherchenort | Abschlußdatum der Recherche | Prüfer |
|---|---|---|
| München | 12. Januar 2006 | Villa Riva, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

**EUROPÄISCHER RECHERCHENBERICHT**

Nummer der Anmeldung

EP 05 01 7958

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 6 685 964 B1 (BARTHOLOMAEUS JOHANNES ET AL) 3. Februar 2004 (2004-02-03) <br> * Spalte 1, Zeile 62 - Spalte 2, Zeile 38 * <br> ----- | 1-22 | |
| X | GAZZANIGA A ET AL: "Oral delayed-release system for colonic specific delivery" INTERNATIONAL JOURNAL OF PHARMACEUTICS (AMSTERDAM), Bd. 108, Nr. 1, 1994, Seiten 77-83, XP002362479 ISSN: 0378-5173 <br> * Seite 78, linke Spalte, Zeilen 14-35 * <br> * Abbildungen 1-4 * <br> ----- | 1-19,21, 22 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 12. Januar 2006 | Villa Riva, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 05 01 7958

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-01-2006

| Im Recherchenbericht angeführtes Patentdokument | Datum der Veröffentlichung | Mitglied(er) der Patentfamilie | Datum der Veröffentlichung |
|---|---|---|---|
| US 6610323  B1 | 26-08-2003 | AT        252379 T | 15-11-2003 |
| | | AU        759311 B2 | 10-04-2003 |
| | | AU       1991399 A | 12-07-1999 |
| | | BG        104619 A | 30-04-2001 |
| | | BR       9814389 A | 10-10-2000 |
| | | CA       2315927 A1 | 01-07-1999 |
| | | CN       1284868 A | 21-02-2001 |
| | | DE      69819205 D1 | 27-11-2003 |
| | | DE      69819205 T2 | 26-08-2004 |
| | | DK       1043977 T3 | 09-02-2004 |
| | | EE     200000395 A | 15-02-2002 |
| | | EP       1043977 A1 | 18-10-2000 |
| | | ES       2209240 T3 | 16-06-2004 |
| | | HR      20000381 A1 | 31-12-2000 |
| | | HU       0200618 A2 | 29-07-2002 |
| | | ID         27040 A | 22-02-2001 |
| | | IL        136823 A | 31-08-2005 |
| | | JP    2001526213 T | 18-12-2001 |
| | | MA        26577 A1 | 20-12-2004 |
| | | NO      20003219 A | 22-08-2000 |
| | | NZ        505135 A | 28-06-2002 |
| | | PL        341565 A1 | 23-04-2001 |
| | | PT       1043977 T | 31-03-2004 |
| | | RU       2205028 C2 | 27-05-2003 |
| | | WO       9932093 A1 | 01-07-1999 |
| | | SK       8082000 A3 | 12-02-2001 |
| | | TR     200001982 T2 | 21-02-2001 |
| | | UA         69396 C2 | 15-11-2000 |
| | | ZA       9811234 A | 22-06-1999 |
| US 5766623  A | 16-06-1998 | KEINE | |
| EP 1419766  A | 19-05-2004 | EP       1475085 A1 | 10-11-2004 |
| | | EP       1477162 A1 | 17-11-2004 |
| | | EP       1504757 A2 | 09-02-2005 |
| | | EP       1502591 A2 | 02-02-2005 |
| | | EP       1504758 A2 | 09-02-2005 |
| EP 1243269  A | 25-09-2002 | KEINE | |
| EP 0572942  A | 08-12-1993 | AT        151285 T | 15-04-1997 |
| | | DE      69309540 D1 | 15-05-1997 |
| | | DE      69309540 T2 | 04-09-1997 |
| | | DK        572942 T3 | 20-10-1997 |
| | | ES       2101164 T3 | 01-07-1997 |
| | | GR       3023605 T3 | 29-08-1997 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 05 01 7958

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-01-2006

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| EP 0572942 | A | | | GR | 3035415 | T3 | 31-05-2001 |
| | | | | HU | 75158 | A2 | 28-04-1997 |
| | | | | IT | 1260505 | B | 09-04-1996 |
| US 5891474 | A | | 06-04-1999 | AU | 5675598 | A | 18-08-1998 |
| | | | | DE | 69731847 | D1 | 05-01-2005 |
| | | | | EP | 0954293 | A1 | 10-11-1999 |
| | | | | ES | 2232886 | T3 | 01-06-2005 |
| | | | | WO | 9832426 | A1 | 30-07-1998 |
| | | | | JP | 2001509157 | T | 10-07-2001 |
| | | | | PT | 954293 | T | 29-04-2005 |
| | | | | US | 6190692 | B1 | 20-02-2001 |
| US 6685964 | B1 | | 03-02-2004 | AT | 257012 | T | 15-01-2004 |
| | | | | AU | 771064 | B2 | 11-03-2004 |
| | | | | AU | 1011300 | A | 20-07-2000 |
| | | | | CA | 2295471 | A1 | 18-07-2000 |
| | | | | CN | 1270029 | A | 18-10-2000 |
| | | | | DE | 19901687 | A1 | 20-07-2000 |
| | | | | DK | 1020185 | T3 | 16-02-2004 |
| | | | | EP | 1020185 | A2 | 19-07-2000 |
| | | | | ES | 2213971 | T3 | 01-09-2004 |
| | | | | HK | 1029749 | A1 | 24-09-2004 |
| | | | | HU | 0000138 | A2 | 28-02-2001 |
| | | | | IL | 134076 | A | 17-05-2005 |
| | | | | JP | 2000212072 | A | 02-08-2000 |
| | | | | NZ | 502261 | A | 30-11-2001 |
| | | | | PL | 337867 | A1 | 31-07-2000 |
| | | | | PT | 1020185 | T | 31-05-2004 |
| | | | | RU | 2239417 | C2 | 10-11-2004 |
| | | | | SK | 642000 | A3 | 14-08-2000 |
| | | | | ZA | 200000173 | A | 07-08-2000 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1243269 A
- EP 1419766 A